(19) Europäisches Patentamt
European Patent Office
Office européen des brevets

(11) **EP 4 635 471 A1**

(12) **DEMANDE DE BREVET EUROPEEN**

(43) Date de publication:
**22.10.2025 Bulletin 2025/43**

(21) Numéro de dépôt: **25169666.2**

(22) Date de dépôt: **10.04.2025**

(51) Classification Internationale des Brevets (IPC):
*A61K 8/25* (2006.01)    *A61K 8/26* (2006.01)
*A61K 8/365* (2006.01)    *A61K 8/73* (2006.01)
*A61Q 19/00* (2006.01)    *A61K 8/92* (2006.01)
*A61K 8/06* (2006.01)

(52) Classification Coopérative des Brevets (CPC):
**A61K 8/922; A61K 8/062; A61K 8/25;
A61K 8/9789; A61Q 19/00;** A61K 2800/78

(84) Etats contractants désignés:
**AL AT BE BG CH CY CZ DE DK EE ES FI FR GB
GR HR HU IE IS IT LI LT LU LV MC ME MK MT NL
NO PL PT RO RS SE SI SK SM TR**
Etats d'extension désignés:
**BA**
Etats de validation désignés:
**GE KH LA MA MD TN**

(30) Priorité: **15.04.2024 FR 2403896**

(71) Demandeur: **Ahaituki SAS**
**56640 Arzon (FR)**

(72) Inventeur: **OMNES, Louis-François**
**75005 PARIS (FR)**

(74) Mandataire: **Eveillard, Sophie**
**Apropis**
**44, rue César**
**56100 Lorient (FR)**

(54) **COMPOSITION COMPRENANT UN MACÉRAT HUILEUX DE FLEURS DE CHANVRE ET UNE ÉMULSION DE PICKERING, PRÉPARATION ET UTILISATIONS**

(57) Composition comprenant une solution contenant au moins un dérivé du chanvre et une émulsion de Pickering H/E, ledit dérivé du chanvre étant choisi parmi les macérats huileux de fleurs de chanvre, les cannabinoïdes, les terpènes et les flavonoïdes et l'émulsion de Pickering H/E contenant une phase aqueuse, une phase huileuse végétale et un phyllosilicate organomodifié, procédé de préparation de la composition et utilisation en tant qu'agent apaisant, relaxant, anti-stress, de la peau.

**EP 4 635 471 A1**

**Description**

La présente invention se rapporte au domaine des compositions à base de dérivés du chanvre et en particulier à base d'huile(s) de fleurs de chanvre. Plus spécifiquement l'invention concerne une composition comprenant au moins un macérat huileux de fleurs de chanvre ainsi que son utilisation en cosmétique en application sur la peau notamment en tant qu'agent anti-stress, apaisant, relaxant, de la peau.

La peau est la principale barrière de protection du corps humain à l'égard des agressions extérieures telles que la pollution atmosphérique, les variations climatiques et le rayonnement UV.

La peau est constituée de trois couches : l'épiderme, le derme et l'hypoderme. L'épiderme, qui est en contact la couche superficielle, est plus particulièrement concerné par les interactions avec le milieu extérieur. L'épiderme est recouvert par un film hydrolipidique et est composé de quatre couches : la couche cornée, la couche granuleuse, la couche épineuse et la couche basale.

La demande de brevet WO2016/123475 décrit une composition comprenant un extrait d'huile de Cannabis et de la vitamine E. Dans le paragraphe consacré à l'art antérieur, les auteurs soulignent le caractère visceux des huiles de cannabis ainsi que les difficultés qui en découlent en particulier lorsqu'on souhaite les utiliser en les vaporisant.

La viscosité des huiles de cannabis est donc un paramètre à prendre en considération lorsqu'on souhaite mettre au point une composition présentant une galénique particulière adaptée à une application sur la peau.

**Résumé de l'invention** L'inventeur a montré qu'une solution au problème technique de l'obtention de nouvelles compositions cosmétiques apaisantes pouvait être apportée au moyen d'une composition comprenant une solution contenant au moins un dérivé du chanvre et une émulsion de Pickering H/E, ledit dérivé du chanvre étant choisi parmi les macérats huileux de fleurs de chanvre, les cannabinoïdes, les terpènes et les flavonoïdes et l'émulsion de Pickering H/E contenant une phase aqueuse, une phase huileuse végétale et un phyllosilicate organomodifié. En particulier la composition selon l'invention comprend au moins un macérat huileux de fleurs de chanvre et une émulsion de Pickering.

Le chanvre ou Cannabis est une plante herbacée à fleurs de la famille des Cannabinacées. Cette plante annuelle, originaire d'Asie centrale ou d'Asie du Sud, présente plusieurs phénotypes pouvant être décrits comme sous-espèces et variétés.

Le chanvre contient plus de 540 principes actifs aujourd'hui identifiés, appartenant à diverses classes chimiques, dont notamment les cannabinoïdes, les terpènes et les flavonoïdes (1).

L'exploitation des différentes parties de la plante permet de répondre à de multiples utilisations industrielles. Ce sont les fleurs féminisées qui sont principalement exploitées dans le secteur médical, du bien-être et de la cosmétique, en particulier en vue d'en extraire deux molécules de la famille des cannabinoïdes : le $\Delta$-9-tétrahydrocannabinol, aussi nommé plus simplerment « tétrahydrocannabinol » ou THC et le Cannabidiol ou CBD.

La communauté scientifique a aujourd'hui établi l'existence d'un « effet entourage » pour le Cannabis, c'est-à-dire une interaction et une synergie des composés d'intérêt contenus dans la plante permettant d'aboutir à des produits au profil bénéfice-risque optimisé (2,3). Une attention nouvelle est donc aujourd'hui portée à de nouvelles molécules issues de la famille des terpènes, des flavonoïdes, mais également d'autres cannabinoïdes comme le Cannabigérol ou CBG.

L'« effet entourage » interroge sur la pertinence des stratégies historiques de développement des industries du chanvre pré-citées exploitant quasi-exclusivement une à deux molécules, THC et CBD, utilisées seules ou en association.

De manière surprenante et avantageuse, l'inventeur a mis en évidence une composition particulière présentant des effets avantageux en cosmétique en application sur la peau, plus particulièrement en tant qu'agent apaisant, relaxant, anti-stress.

Selon un premier aspect, la présente invention vise une composition comprenant une solution contenant au moins un dérivé du chanvre et une émulsion de Pickering H/E, ledit dérivé du chanvre étant choisi parmi les macérats huileux de fleurs de chanvre, les cannabinoïdes, les terpènes et les flavonoïdes et l'émulsion de Pickering H/E contenant une phase aqueuse, une phase huileuse végétale et un phyllosilicate organomodifié.

Selon un deuxième aspect, la présente invention vise une composition comprenant une solution contenant au moins un macérat huileux de fleurs de chanvre et une émulsion de Pickering H/E contenant une phase aqueuse, une phase huileuse végétale et un phyllosilicate organomodifié.

De préférence, la solution contenant au moins un macérat huileux de fleurs de chanvre est présente en une teneur allant de 0,1 à 25%, de préférence allant de 1% à 5% et avantageusement 3,4% en poids par rapport au poids total de la composition.

La présente invention vise encore un procédé de préparation de la composition selon l'invention.

La présente invention vise également l'utilisation cosmétique, non thérapeutique de la composition selon l'invention en tant qu'agent apaisant, relaxant, anti-stress, de la peau.

La présente invention vise aussi un procédé de traitement cosmétique, non thérapeutique de la peau, comprenant au moins une étape d'application sur la peau de la composition selon l'invention.

Ces compositions cosmétiques sont en outre stables, non irritantes, non toxiques, non allergisantes pour la peau.

D'autres aspects, avantages, propriétés de la présente invention sont présentés dans la description et les exemples qui suivent.

## Description détaillée

### Définitions

Dans le présent texte, sauf indications contraires spécifiques, les pourcentages sont exprimés en poids d'une composition de référence.

Dans le présent texte, les intervalles sont définis de façon abrégée afin d'éviter de décrire chacune des valeurs et toutes les valeurs de cet intervalle, cependant toute valeur appropriée dans l'intervalle peut être choisie comme la valeur supérieure, la valeur inférieure ou les valeurs terminales de l'intervalle. Par exemple, un intervalle de 0,1 à 1,0 représente les valeurs terminales de 0,1 et 1,0, ainsi que les valeurs intermédiaires de 0,2 ; 0,3 ; 0,4 ; 0,5 ; 0,6 ; 0,7 ; 0,8 ; 0,9 et tous les intervalles intermédiaires compris dans 0,1 à 1,0, tels que 0,2 à 0,5 ; 0,2 à 0,8 ; 0,7 à 1,0... Sauf indications contraires, un intervalle défini comme étant « compris entre la valeur A et la valeur B » inclut les valeurs A et B et est donc équivalent à un intervalle « allant de la valeur A à la valeur B » , l'expression « au moins » comprend la valeur énoncée après, par exemple, «au moins 5 % » doit être compris comme comprenant également « 5 % », l'expression « un maximum de » comprend la valeur énoncée après, par exemple, « un maximum de 5 % » doit être compris comme comprenant également « 5 % ».

En outre, dans le présent texte, les valeurs mesurables, telles qu'une quantité, doivent être comprises comme comprenant les écarts types qui peuvent être facilement déterminés par l'homme du métier dans le domaine technique de référence. De préférence, ces valeurs sont destinées à comprendre des variations de $\pm$ 5 %.

Dans le présent texte, les mots « chanvre » et « Cannabis » sont utilisés de manière équivalente.

Par « peau » on entend l'épiderme du visage ou du corps ou du cuir chevelu.

Les compositions selon la présente invention sont des compositions cosmétiques, non thérapeutiques. Les compositions cosmétiques sont destinées à être appliquées sur les peaux saines, par simple massage pour traiter l'épiderme. Elles sont conformes au règlement CE 1223/2009.

### Macérat(s) huileux de chanvre

La solution contenant au moins un macérat huileux de fleurs de chanvre utilisable selon l'invention est une solution comprenant au moins 50%, de préférence au moins 75% en poids d'un macérat huileux obtenu par macération de fleurs de chanvre dans une huile de graines de chanvre.

De préférence ce macérat est obtenu par macération de 500 à 1000g de fleurs de chanvre pour 1 L d'huile de graines de chanvre.

Le ratio masse de fleurs de chanvre / huile de graines de chanvre est avantageusement compris entre compris entre 1/3 et 3/1 et de manière préférée entre 1/2 et 1/1.

En général, la durée de la macération, encore appelée « infusion » est de 3 à 4 semaines. Il relève des compétences de l'homme du métier de définir la durée de la macération.

Toutes les fleurs de chanvre sont utilisables, sous réserve, bien entendu, que leur utilisation soit autorisée par les règlementations nationales ou transnationales considérées.

En Europe, c'est notamment le cas des souches à haute teneur en CBD issues du Catalogue Commun des Variétés des Espèces de Plantes Agricoles, telles que la Kompolti, la Carmagola, la Perugina, la Finola, ou plus récemment la Pain Killer, la Midwest ou la Strawberry K. Dans d'autres pays, notamment américains, d'autres souches génétiques d'intérêt peuvent être utilisées, telles que par exemple l'ACDC, la Charlotte's web, l'Harlequin, la Harle-Tsu, la Ringo's gift.

Avantageusement les fleurs utilisées sont manucurées, c'est-à-dire que l'inflorescence a été récoltée à la main afin de préserver au maximum la qualité du totum de la fleur.

A ce macérat principal peut être ajouté un autre macérat, secondaire, de fleurs de chanvre dans une huile végétale différente de l'huile de graines de chanvre, par exemple l'huile de tournesol.

L'ajout d'un second macérat dans lequel d'autres fleurs de chanvre ont été décarboxylées, permet de garantir un meilleur « effet entourage » de la solution selon l'invention, c'est-à-dire de garantir qu'une plus grande variété de composés des fleurs de chanvre, avec leurs propriétés cosmétiques spécifiques, soient présents dans la solution.

La décarboxylation consiste à chauffer un macérat huileux pour convertir les cannabinoïdes de leur forme acide en leur forme active, la décarboxylation peut aussi être obtenue par l'action des UV sur le macérat. Ce processus convertit notamment le CBDA (Cannabidiol-Acide) en CBD et le THCA (Tétrahydrocannabinol-Acide) en THC.

Avantageusement le ratio en volume (v/v) entre le macérat huileux obtenu par macération de fleurs de chanvre dans une huile de graines de chanvre (macérat principal) et le macérat huileux obtenu par macération de fleurs de chanvre dans une huile différente (macérat secondaire) est strictement supérieur à 1/1, de préférence il est de 3/1.

De manière préférée, la solution contenant au moins un macérat huileux de fleurs de chanvre comprend au moins du CBD en une quantité allant de 0,005% à 24,000%, de préférence de 0,010% à 5,000% en poids par rapport au poids total de la solution; du CBDA en une quantité allant de 0,005% à 24,000%, de préférence 0,010% à 5,000% en poids par rapport au poids total de la solution; du THC en une quantité allant de 0,001% à 30,000%, de préférence 0,010% à 1,000% en poids par rapport au poids total de la solution; du CBG en une quantité allant de 0,001% à 22,000%, de préférence 0,010% à 3,000% en poids par rapport au poids total de la solution et du CBGA en une quantité allant de 0,001% à 22,000%, de préférence 0,010% à 3,000% en poids par rapport au poids total de la solution.

Bien entendu, ces quantités seront adaptées aux règlementations nationales ou transnationales du/des pays où l'exploitation est envisagée.

**Emulsions de Pickering**

Les émulsions de Pickering sont des émulsions stabilisées par des particules solides. Lors de la préparation de l'émulsion, lesdites particules solides se positionnent à l'interface entre la phase aqueuse et la phase huileuse.

Les émulsions de Pickering selon la présente invention sont des émulsions huile dans eau, c'est-à-dire H/E, stabilisées par une argile particulière qui est un phyllosilicate naturel modifié.

Avantageusement, la phase aqueuse de l'émulsion de Pickering est présente en une quantité allant de 51% à 90%, de préférence 55% à 70% et de manière préférée 58% à 65% en poids par rapport au poids total de la composition.

Selon un mode de réalisation préféré, le phyllosilicate naturel modifié comprend un phyllosilicate choisi dans le groupe constitué par les vermiculites et les smectites. De préférence, le phyllosilicate peut être choisi dans le groupe constitué par les montmorillonites, bentonites, nonytronites, beidellites, volkonskoites, hectorites, saponites, sauconites, sobockites, stevensites, svinfordites, de préférence ce sont des phyllosilicates de sodium, de potassium, de calcium, ou de leurs mélanges. De manière plus préférée, le phyllosilicate est choisi dans le groupe constitué par l'hectorite, la montmorillonite, la bentonite ou leurs mélanges.

Selon un mode de réalisation préféré, le phyllosilicate naturel modifié comprend un composé organique choisi dans le groupe constitué par la gomme xanthane, la gomme guar, la gomme de Tara ou de Caroube, la gomme d'Acacia, le carraghénane, l'alginate, le chitosane, la pectine, l'acide citrique, l'acide tartrique, l'acide oxalique, l'acide succinique, l'acide malique, l'acide acétique, l'acide lactique, l'acide propionique, l'acide salicylique, les glycosaminoglycanes. Avantageusement, le phyllosilicate naturel modifié comprend une bentonite modifiée par la gomme de xanthane et l'acide citrique.

Selon un autre mode de réalisation, le phyllosilicate naturel modifié comprend un composé organique choisi parmi tout composé organique connu dans l'art des émulsions de Pickering, tels que ceux divulgués dans le brevet français n° FR 2,976,503.

Avantageusement, le phyllosilicate modifié est présent en une quantité allant de 3% à 20%, de préférence 4% à 10% et de manière préférée 5% à 8% en poids par rapport au poids total de la composition.

Selon un mode de réalisation préféré, la phase huileuse vegétale de l'émulsion de Pickering comprend au moins une huile végétale choisie dans le groupe formé par l'huile de chanvre, l'huile de tournesol, l'huile de colza, l'huile d'olive, l'huile de cameline, l'huile d'arachide, l'huile de coco, l'huile de pépin de raisin, l'huile de ricin, l'huile d'argan, l'huile de Djansang, l'huile de dattier du désert, l'huile de nigelle, l'huile de figue de barbarie, l'huile de macadamia, l'huile de soja, l'huile de palme et de palmier, l'huile de Tamanu, l'huile de sésame, l'huile de lin, l'huile de noix, l'huile de noisette, l'huile de baobab, l'huile de fruit de la passion, l'huile de noix du Brésil, l'huile d'hibiscus, l'huile de pépin de courge, l'huile de Luffa, l'huile de Carapa, l'huile d'Onagre, l'huile de bourrache, huile d'avocat, l'huile d'amande, l'huile d'argousier, l'huile de noyau d'abricot, l'huile de noyau de cerise, l'huile de pépin de pomme, l'huile de grenade, l'huile de Jojoba, l'huile de rose musquée, l'huile de prune, le beurre de karité, le beurre de cacao, le beurre de Kokum, le beurre de mangue, le beurre de moabi, le beurre de Karanja, le beurre de Tucuma, le beurre de Cupuaçu, le beurre de Buriti, le beurre de Murumuru, le beurre de Kombo, le beurre de Kpangnan, les triglycérides d'acides caprylique/caprique.

Avantageusement, la phase huileuse de l'émulsion de Pickering est présente en une quantité allant de 5% à 40%, de préférence 10% à 30% et de manière préférée 15% à 25% en poids par rapport au poids total de la composition.

**Composition**

Avantageusement la composition selon la présente invention comprend, dans un milieu acceptable, au moins un agent cosmétique choisi parmi les agents humectants, les épaississants, les agents de texture, les émulsifiants, les agents dispersants, les agents moussants, les émolients, les conservateurs, les colorants, les extraits de plantes, les fibres végétales, les minéraux, les agents correcteurs de pH, les principes actifs et les parfums.

Avantageusement la composition selon la présente invention comprend de 0,001 à 20 % en poids d'au moins un agent cosmétique par rapport au poids total de la composition.

Bien entendu, l'homme du métier veillera à choisir ces agents cosmétiques de manière à ne pas altérer les propriétés de la composition selon l'invention.

La composition selon l'invention peut constituer une composition de massage, de soin de la peau, et notamment une crème de nettoyage, de protection, de traitement ou de soin pour le visage, pour les mains, pour les pieds, ou pour le corps, en particulier la composition peut être une crème de jour, crème de nuit, crème démaquillante, crème de fond de teint, crème antisolaire ; un fond de teint fluide, un lait de démaquillage, un lait corporel de protection ou de soin, un lait anti-solaire; une lotion, gel ou mousse pour le soin de la peau, comme une lotion micellaire de nettoyage.

De manière avantageuse, le procédé de préparation de la composition selon l'invention comprend au moins les étapes suivantes dans cet ordre : préparer la phase aqueuse ;

ajouter le phyllosilicate organomodifié à la phase aqueuse et mélanger ; ajouter la phase huileuse végétale au mélange obtenu et obtenir une émulsion de Pickering H/E ;
ajouter la solution contenant au moins un macérat huileux de fleurs de chanvre à l'émulsion de Pickering H/E.

Bien entendu, la phase huileuse végétale de l'émulsion de Pickering est différente de la solution contenant au moins un macérat huileux de fleurs de chanvre.

Il a été noté que les viscosités des macérats huileux de fleurs de chanvre sont compaptibles avec le procédé de préparation des compositions selon l'invention.

**Utilisations**

La composition cosmétique selon l'invention est avantageusement destinée à être appliquée sur la peau.

La présente invention vise encore l'utilisation cosmétique, non thérapeutique, de la composition selon l'invention en tant qu'agent apaisant, relaxant, anti-stress de la peau. Elle vise aussi un procédé de traitement cosmétique, non thérapeutique de la peau, comprenant au moins une étape d'application sur la peau de la composition selon l'invention.

Les exemples qui suivent visent à illustrer l'invention sans en limiter la portée.

**Exemples**

**A. Macérats**

**Exemple A1- macérat 1**

Dans le tableau 1 sont répertoriés les produits utilisés pour préparer les macérats

[Tableau 1]

| Produit | Quantité | Fournisseur |
|---|---|---|
| Huile de graines de chanvre Bio 1ère pression à froid | 7.5 L | ABCD (France) |
| Huile de tournesol désaromatisée | 2.5 L | (Bouton D'or Bio) France |
| Chanvre Kompolti - Fleurs manucurées | 5000 g | ABCD (France) |
| Chanvre Carmagnola - Fleurs manucurées | 2000 g | ABCD (France) |
| Chanvre Perugina - Fleurs manucurées (sous serre) | 1000 g | ABCD (France) |

Les fleurs de chanvre utilisées sont toutes des fleurs sèches manucurées puis broyées avec un seuil de coupure à 250 microns.

Elles sont ensuite mélangées pour former un mix de fleurs de chanvre broyées.

Préparation

Dans un premier réacteur ont été introduits les 2.5 L d'huile de tournesol désaromatisée et 2000 g de mix de fleurs broyées. Ce mélange est ensuite chauffé au bain marie, à 95°C, pendant 1 heure en vue de la décarboxylation de certains composants actifs (transformation du CBA en CBD, du CBGA en CBG et du THCA en THC).

Cette huile « décarboxylée » est ensuite filtrée.

Dans un deuxième réacteur ont été introduits les 7.5 L d'huile de graines de chanvre et 6000 g de mix de fleurs broyées. Ce mélange est ensuite laissé macérer pendant 3 semaines. Durant ces 3 semaines, le macérat est remué tous les jours pendant 3 minutes.

Le macérat obtenu dans le deuxième réacteur est ensuite laissé décanter pendant 3 jours c'est-à dire qu'il n'est pas remué. Il est ensuite filtré au moyen d'un filtre à café. Les fleurs restant dans le fond de la cuve de décantation sont récupérées et pressées au moyen d'une presse à huile ECOLEA.

L'huile de décantation, l'huile de pressage de fond de cuve -issues du deuxième réacteur- et l'huile de tournesol -issue du premier réacteur- sont réunies. L'huile obtenue est nommée Macérat 1.

L'analyse HPLC, avec un DAD (détecteur à barrette de diodes) Shimadzu, du macérat 1 a permis de déterminer les teneurs suivantes consignées dans le tableau 2 :

[Tableau 2]

| Composés | % masse |
|----------|---------|
| CBD | 0.188 |
| CBDA | 0.543 |
| THC | 0.024 |
| CBG | 0.096 |
| CBGA | 0.553 |

Soit une teneur en CBD total de 0.664% et une teneur en CBG total de 0.582%. Pour obtenir ces valeurs, les formes acides (CBDA et CBGA) ont été multipliées par un facteur de 0.878.

**Exemple A2- macérat 2**

Dans le tableau 3 sont répertoriés les produits utilisés pour préparer les macérats

[Tableau 3]

| Produit | Quantité | Fournisseur |
|---------|----------|-------------|
| Huile de graines de chanvre Bio 1ère pression à froid | 7.5 L | ABCD (France) |
| Huile de tournesol désaromatisée | 2.5 L | (Bouton D'or Bio) France |
| Chanvre Kompolti - Fleurs manucurées | 3000 g | ABCD (France) |
| Chanvre Carmagnola - Fleurs manucurées | 1000 g | ABCD (France) |
| Chanvre Perugina - Fleurs manucurées (sous serre) | 1000 g | ABCD (France) |
| Trichome de Kompolti (poudre issue de fleurs broyées à 250 $\mu$m dans l'huile d'olive) | 500 g | ABCD (France) |

Les fleurs de chanvre utilisées sont toutes des fleurs sèches manucurées puis broyées avec un seuil de coupure à 250 microns.

Elles sont ensuite mélangées pour former un mix de fleurs de chanvre broyées.

Préparation

Dans un premier réacteur ont été introduits les 2.5 L d'huile de tournesol désaromatisée et 1250 g de mix de fleurs broyées. Ce mélange a ensuite été chauffé à feu doux, à 125°C, pendant 30 minutes en vue de la décarboxylation de certains composants actifs (transformation du CBA en CBD, du CBGA en CBG et du THCA en THC). Cette huile décarboxylée est ensuite filtrée.

Dans un deuxième réacteur ont été introduits les 7.5 L d'huile de chanvre, 3750 g de mix de fleurs broyées et les 500 g de Trichome de Kompolti. Ce mélange est ensuite laissé macérer pendant 3 semaines. Durant ces 3 semaines, le macérat est remué tous les jours pendant 3 minutes.

Le macérat obtenu dans le deuxième réacteur est ensuite laissé décanter pendant 3 jours c'est-à dire qu'il n'est pas remué. Il est ensuite filtré au moyen d'un filtre à café.

L'huile de décantation -issue du deuxième réacteur- et l'huile de tournesol -issue du premier réacteur- sont réunies. L'huile obtenue est nommée Macérat 2.

L'analyse HPLC, avec un DAD Vanquish Thermo Scientific, du macérat 2 a permis de déterminer les teneurs suivantes consignées dans le tableau 4 :

[Tableau 4]

| Composés | % masse |
|---|---|
| CBD | 2.025 |
| CBDA | 0.596 |
| THC | 0.032 |
| CBG | 0.029 |
| CBGA | 0.239 |

Soit une teneur en CBD total de 2,548% et une teneur en CBG total de 0.239%. Pour obtenir ces valeurs, les formes acides (CBDA et CBGA) ont été multipliées par un facteur de 0.878.

**Exemple A3- macérat 3**

Dans le tableau 5 sont répertoriés les produits utilisés pour préparer les macérats

[Tableau 5]

| Produit | Quantité | Fournisseur |
|---|---|---|
| Huile de graines de chanvre Bio 1ère pression à froid | 10.0 L | ABCD (France) |
| Chanvre Kompolti - Fleurs manucurées | 5000 g | ABCD (France) |
| Chanvre Carmagnola - Fleurs manucurées | 1000 g | ABCD (France) |

Les fleurs de chanvre utilisées sont toutes des fleurs sèches manucurées puis broyées avec un seuil de coupure à 250 microns.

2500 g de fleurs Kompolti et les 1000 g de fleurs Carmagnola sont broyées et mélangées pour former un mix de fleurs de chanvre broyées.

Préparation

Les 2500 g de fleurs Kompolti restantes sont chauffées au four, à 120°C, pendant 20 minutes. Le traitement conduit à décarboxyler les fleurs Kompolti.

Dans un réacteur ont été introduits les 10.0 L d'huile de chanvre, les 2500 g de fleurs Kompolti décarboxylées et les 3500 g de mix de fleurs de chanvre broyées. Ce mélange est ensuite laissé macérer pendant 3 semaines. Durant ces 3 semaines, le macérat est remué tous les jours pendant 3 minutes.

Le macérat obtenu est ensuite filtré au moyen d'un filtre à café

L'huile obtenue est nommée Macérat 3.

L'analyse HPLC, avec un DAD Vanquish Thermo Scientific, du macérat 3 a permis de déterminer les teneurs suivantes consignées dans le tableau 6.

[Tableau 6]

| Composés | % masse |
|---|---|
| CBD | 1.044 |
| CBDA | 0.521 |
| THC | 0.033 |
| CBG | 0.030 |
| CBGA | 0.084 |

Soit une teneur en CBD total de 1.502 % et une teneur en CBG total de 0.103%. Pour obtenir ces valeurs, les formes acides (CBDA et CBGA) ont été multipliées par un facteur de 0.878.

Il a été vérifié que les macérats 1, 2 et 3 sont similaires vis-à-vis des propriétés apaisantes visées.

**B. Compositions testées**

1- Composition comparative **C1**

La composition comparative **C1** correspond au macérat M1.

2- Composition comparative **C2**

La composition comparative **C2** comprend une émulsion de Pickering H/E contenant une argile qui est un phyllosilicate modifié (sans macérat huileux de chanvre).

Dans le tableau 7 sont répertoriés les produits utilisés pour préparer la composition **C2.**

[Tableau 7]

| Phase | Nom commercial | % | INCI |
|---|---|---|---|
| A | Eau | 73,70 | Aqua |
| B | Georgard Ultra commercialisé par Lonza | 1,00 | Gluconolactone & Sodium benzoate & Calcium gluconate |
| B | Benzoate de Sodium | 0,30 | Sodium benzoate |
| C | Frametime CXG commercialisé par Ephyla | 4,50 | Bentonite & xanthan gum & Sodium stearoyl glutamate & citric acid |
| C | Gommme de xanthane FF commercialisée par Jungbunzlauer | 0,50 | xanthan gum |
| D | Huile de tournesol raffinée commercialisée par CAUVIN | 20,00 | Helianthus annuus seed oil |
| Total | | 100 | |

Préparation de la composition **C2** :

Les phases A et B sont mélangées pour former une phase aqueuse puis la phase C est ajoutée et le tout est mélangé. Enfin la phase huileuse D est ajoutée et le tout est mélangé. Une fois l'émulsion de Pickering obtenue, le pH est ajusté à 5,00.

3- Composition **C3**

La composition comparative **C3** comprend un macérat et une émulsion de Pickering H/E contenant un kaolin.

Dans le tableau 8 sont répertoriés les produits utilisés pour préparer la composition **C3.**

[Tableau 8]

| Phase | Nom commercial | % | INCI |
|---|---|---|---|
| A | Eau | 70,30 | Aqua |
| B | Georgard Ultra commercialisé par Lonza | 1,00 | Gluconolactone & Sodium benzoate & Calcium gluconate |
| B | Benzoate de Sodium | 0,30 | Sodium benzoate |
| C | Argile blanche commercialisée par COOPER | 4,50 | Kaolin |
| C | Gommme de xanthane FF commercialisée par Jungbunzlauer | 0,50 | xanthan gum |
| D | Huile de tournesol raffinée commercialisée par Cauvin | 20,00 | Helianthus annuus seed oil |
| E | Macérat 1 | 3,40 | |

(suite)

| Phase | Nom commercial | % | INCI |
|-------|----------------|-----|------|
| Total | | 100 | |

Préparation de la composition **C3** :

Les phases A et B sont mélangées pour former une phase aqueuse puis la phase C est ajoutée et le tout est mélangé. Enfin la phase huileuse D est ajoutée et le tout est mélangé.

Une fois l'émulsion de Pickering obtenue, la phase E c'est-à- dire le macérat 1 est ajouté et le tout est mélangé. Enfin, le pH est ajusté à 5,00.

4- Composition **I1** conforme à l'invention

La composition conforme **I1** comprend un macérat et une émulsion de Pickering H/E contenant une argile qui est un phyllosilicate modifié.

Dans le tableau 9 sont répertoriés les produits utilisés pour préparer la composition **I1.**

[Tableau 9]

| Phase | Nom commercial | % | INCI |
|-------|----------------|-----|------|
| A | Eau | qsp | Aqua |
| B | Frametime CX commercialisé par Ephyla | 6,00 | Bentonite & xanthan gum & citric acid |
| B | Amisoft Hs11p | 0,30 | Sodium stearoyl glutamate |
| B | Gommme de xanthane FF commercialisée par Jungbunzlauer | 0,50 | Xanthan gum |
| C | Alcool cetosréarylique 50/50 | 4,00 | Cetearyl alcohol |
| C | Ephyster MCR | 20,00 | Brassica napus extract |
| C | HTR1 commercialisé par EPHYLA | 1,50 | Helianthus annuus seed oil & Protium heptaphyllum resin |
| C | Sepicide LD | 1,00 | Phenoxyethanol |
| D | Macerat 1 | 3,40 | |
| total | | 100,00 | |

Préparation de la composition **I1**

Les phases A et B sont mélangées pour former une phase aqueuse puis la phase C est ajoutée et le tout est mélangé.

Une fois l'émulsion de Pickering obtenue, la phase D c'est-à- dire le macérat 1 est ajouté et le tout est mélangé. Enfin, le pH est ajusté à 5,00.

**C. Activité sur une enzyme de la peau dans un modèle *in Vitro***

Des tests ont été réalisés *in vitro,* sur l'enzyme clé de la cascade arachidonique : la lipoxygénase, enzyme présente dans l'épiderme de la peau.

Cette étude est basée sur l'influence de produits cosmétiques vis-à-vis de l'activité de l'enzyme Lipoxygénase de sorte à pouvoir comparer et classifier l'influence de ces produits sur cette activité enzymatique. Cette étude vise à illustrer l'impact de la galénique topique sur une activité enzymatique de la peau.

Les mesures de l'activité de l'enzyme lipoxygénase de plusieurs compositions ont été effectuées dans un modélé *in vitro* cellulaire au moyen d'un kit.

*Matériel et Réactifs*

Le kit d'analyse utilisé est le kit vendu sous le nom « Lipoxygenase Inhibitor Screening Assay Kit » par la société CAYMAN/INTERCHIM.

Dans le tableau 10 sont répertoriés les composants du kit.

[Tableau 10]

| TAMPON concentré TRIS-HCL | CAYMAN/INTERCHIM |
|---|---|
| CHROMOGEN 1 = réactif de développement 1 | CAYMAN/INTERCHIM |
| CHROMOGEN 2 = réactif de développement 2 | CAYMAN/INTERCHIM |
| ENZYME : 15-LIPOXYGENASE | CAYMAN/INTERCHIM |
| ACIDE ARACHIDONIQUE | CAYMAN/INTERCHIM |
| HYDROXIDE DE POTASSIUM 0,1 M | CAYMAN/INTERCHIM |
| INHIBITEUR NDGA | CAYMAN/INTERCHIM |

*Spectromètre*

Les mesures ont été effectuées au moyen d'un spectromètre POLARstar Omega de la société BMG LABTECH.

*Préparation des réactifs*

1. R1 : Tampon de test de dépistage des inhibiteurs de la lipoxygénase - Diluer 3 ml de TAMPON concentré avec 27 ml d'eau de qualité HPLC. Le tampon de test R1 (Tris-HCl 0,1 M, pH 7,4) doit être utilisé pour la dilution des échantillons et de l'ENZYME : 15-LIPOXYGENASE (standard 15-LO) avant le dosage. Une fois stocké à 4°C, ce tampon de test R1 est stable pendant au moins deux mois.

R2 Chromogène

Préparez le chromogène R2 avant utilisation en mélangeant des volumes égaux de CHROMOGEN 1 et de CHROMOGEN 2 dans un tube à essai et au vortex. Le volume de Chromogène R2 à préparer est en fonction du nombre de puits analysés (100 µl pour chaque puits). Le Chromogène R2 est à utiliser dans l'heure.

R3 : Étalon de 15-lipoxygénase -

On procéde au transfert de 10 µl de l'ENZYME : 15-LIPOXYGENASE dans un flacon et on dilue avec 990 µl de R1 avant utilisation, conserver sur la glace et utiliser dans l'heure.

R4 Acide arachidonique (substrat) -

Le flacon fourni dans le kit contient une solution d'acide arachidonique (ACIDE ARACHIDONIQUE) dans l'éthanol et doit être conservé à -20°C.

Transférer 25 µl du produit ACIDE ARACHIDONIQUE dans un autre flacon, ajouter 25 µl de la solution d'hydroxyde de potassium, passer au vortex et diluer avec 950 µl d'eau de qualité HPLC pour obtenir la solution R4 à 1 mMUn aliquot de 10 µl donnera une concentration réactionnelle de 91 µM dans les puits.

Le kit contient un flacon d'hydroxyde de potassium (KOH) 0,1 M prêt à l'emploi

R5 Inhibiteur de contrôle positif NDGA -

Le kit contient un flacon de 550 nmoles d'inhibiteur non sélectif de la lipoxygénase acide nordihydroguaiarétique (NDGA).

Remettre en suspension dans 500 µl de R1 pour faire un stock de 1,1 mm. L'ajout de 10 µl au test donne un résultat final concentration de 100 μM d'inhibiteur dans le puits.

- Chaque puits présente un volume final de 210 μl.

*Exécution du test*

1. Puits vierges Echantillons (Blanc Echantillon) :
ajoutez 90 μl de solution R1 et 10 μl du produit à l'essai en triplicate
2. Puits de contrôle positif (activité basale = Témoin +) :
ajoutez 90 μl de solution R3 et 10 μl de solution R1 en triplicate.
3. Puits Produit à l'essai :
ajoutez 90 μl de solution R3 et 10 μl du produit à l'essai en triplicate.
4. Puits d'inhibiteur (Témoin -)
ajoutez 90 μl de solution R3, 10 μl de solution R5 en triplicate;
5. Incuber pendant cinq minutes à température ambiante.
6. Initier la réaction en ajoutant 10 μl de solution R4 dans tous les puits. Placer la plaque 96 puits sur un shaker pendant au moins dix minutes.
7. Ajoutez 100 μl de solution R2 dans chaque puits pour développer la réaction. Couvrir d'un couvercle de plaque et placer la plaque 96 puits sur un shaker pendant cinq minutes.
8. Retirez le couvercle et lisez l'absorbance à 500 nm à l'aide d'un lecteur de plaque.

*Principe de la réaction Système d'essais*

Une solution tamponnée de lipoxygénase (R3) réagit avec un substrat spécifique, l'acide arachidonique (R4), et le transforme pour former un composé qui se lie à un chromogène (R2), sous agitation à température ambiante. L'activité de la lipoxygénase peut ainsi être évaluée par mesure de l'absorbance à 500 nm.

L'échantillon ou le produit de référence : inhibiteur NDGA (R5) est mis en contact avec la solution de lipoxygénase (R3) en même temps que le substrat de l'enzyme (R4). Le substrat (R4) transformé par l'enzyme est coloré à l'aide du chromogène R2 par agitation à température ambiante. L'activité de la lipoxygénase dans les différents puits est alors évaluée par mesure de l'absorbance à 500 nm.

La modulation de cette activité est exprimée en pourcentage d'inhibition ou d'activation de l'activité de la lipoxygénase par rapport au taux basal d'activité en absence d'échantillon ou de produit de référence, c'est-à-dire uniquement en présence du substrat de l'enzyme (acide arachidonique).

*Protocole d'incubation*

Une solution d'enzyme lipoxygénase est incubée dans son substrat, acide arachidonique, pendant 10 minutes, en absence ou présence de l'inhibiteur de référence et de l'échantillon testé puis le chromogène est incorporé avant une incubation de 35 minutes à température ambiante sur agitateur orbital de type Incu-Shaker Mini de la société Benchmark.

*Evaluation des effets*

A la fin de la période d'incubation, l'activité de l'enzyme lipoxygénase avec et sans produit à l'essai ou de référence a été évaluée par mesure de l'absorbance des milieux réactionnels à 500 nm.

Pour chaque concentration testée, la modulation de l'activité de l'enzyme lipoxygénase par le produit à l'essai est calculée selon la formule suivante

Pourcentage de modulation de l'activité de l'enzyme lipoxygénase =

100 x [(DO produit à l'essai– DO lipoxygénase seule (blanc))/DO lipoxygénase seule]

Si le résultat est négatif, le pourcentage est exprimé en inhibition de l'enzyme.

Les résultats en termes de moyenne de DO, moyenne de DO moins le blanc, % d'activité enzymatique sont présentés dans le tableau 11.

[Tableau 11]

| Echantillon | Moyenne DO Ech. | Moyenne DO Ech. - DO blanc | Activité enzyma tique (%) |
|---|---|---|---|
| Blanc | 0,221 | 0,000 | 0,00 |
| T+ | 0,436 | 0,215 | 100,00 |
| T- | 0,187 | -0.034 | 0,00 |
| **C1** | 0,812 | 0,217 | 101,24 |
| **C2** | 1,653 | -0,073 | 0,00 |
| **C3** | 3,845 | 0.054 | 25,00 |
| **I1** | 2,437 | 0,578 | 269,10 |

Le niveau de signification entre le "contrôle" et le "produit de référence" a été évalué à l'aide d'un test *t* de Student (* : p<0,05).

Le niveau de signification entre le le "contrôle" et chaque "composé testé" a été évalué indépendamment pour chaque composition par une analyse de variance à un facteur (ANOVA à sens unique) suivie d'un test de Holm-Sidak (* : p<0,05).

*Conclusions*

La composition selon l'invention **I1** présente une activité enzymatique de 269% (soit +169% par rapport à l'activité normale basale de 100%) alors que la composition comparative **C1** de macérat huileux de chanvre seul présente une activité enzymatique de 101% et la composition comparative **C2** comprenant l'émulsion de Pickering seule présente une activité enzymatique de 0%.

Notons aussi que la composition comparative **C3** comprenant le macérat huileux de chanvre et une émulsion de Pickering à base de kaolin présente une activité enzymatique de seulement 25%.

La composition selon l'invention présente un effet de synergie sur l'activité enzymatique étudiée qui n'est pas observé lorqu'une émulsion de Pickering à base de kaolin est utilisée.

De manière surprenante et inattendue la composition selon l'invention **I1** stimule fortement l'activité enzymatique, alors que la composition **C1** *i.e.* le macérat 1 ne présente pas de modulation du taux basal, et que la composition **C2** (qui constitue le squelette de la composition selon l'invention) sans macérat 1, au contraire inhibe totalement l'activité enzymatique. De manière aussi surprenante la composition **C3** comprenant une émulsion de Pickering réalisée à partir de kaolin montre une inhibition de l'ordre de 75% de l'activité enzymatique par rapport au taux basal (= normal) de l'activité de l'enzyme.

Le macérat 1 **(C1)** seul ne module pas l'activité basale de l'enzyme, tandis que la galénique pickering **(C2)** apporte un effet inhibiteur sur l'activité enzymatique, et qu'au contraire la composition selon l'invention **(11)** apporte une stimulation.

**D- Mesure de l'effet relaxant ou déstressant sur explants de peau humaine par mesure d'IL-6 (sécrétion d'interleukine 6).**

La peau, notamment l'épiderme, sécrète un taux basal de médiateur cellulaire qui reflète à la fois le stress intercellulaire, mais aussi le stress environnemental (c'est une forme d'expression de la réactivité de notre épiderme en contact avec son environnement extérieur).

Cette étude est basée sur l'influence de la composition conforme à l'invention **I1** sur la capacité à moduler la production Interleukine 6.

L'effet recherché est un effet inhibiteur qui se traduit par un effet apaisant ou relaxant ou anti-stress sur l'épiderme qui est en contact avec le milieu extérieur.

*Principe de l'étude*

Pour cette étude des explants cutanés humains frais normaux sont exposés aux échantillons à tester pendant un temps de contact de 6 heures.

A la fin de ce temps de contact, il a été procédé à la dissection et à la mise en suspension des explants dans du

**EP 4 635 471 A1**

sérum physiologique, la concentration en IL-6 des explants est déterminée par dosage Elisa via un kit Enzo IL-6.

*Système d'essai*

Pour cette étude des explants cutanés humains sont exposés aux échantillons à tester avant d'être disposés sur des cellules de Franz ; la mise en place sur cellules de Franz induit un stress environnemental attendu car la peau est pincée et/ou clipsée sur sur les rebords de la cellule de Franz.

Les systèmes explants cutanés/cellules de Franz sont maintenus pendant 6h à 32°C $\pm$ 1°C. A l'issu de l'incubation les explants sont rincés, disséqués et conservés dans du sérum physiologique avant d'être soumis à un cycle d'ultra-sons pendant 30 minutes. Les explants sont alors centrifugés pendant 15 minutes à 5000 rpm et à 4°C puis les surnageants sont conservés et utilisés pour le dosage des interleukines 6.

Les surnageants d'explants cutanés sont utilisés pour le dosage des interleukines 6 à l'aide du kit Enzo IL-6 (human) high sensitivity ELISA Kit. Ce kit fournit un test pratique pour le dosage des interleukines 6 permettant ainsi d'évaluer l'effet modulateur d'actifs ou de produit topique à tester. Ce kit met en œuvre une méthode de dosage immuno-enzymatique pour lequel les échantillons sont mis en contact avec des anticorps monoclonaux spécifiques aux interleukines 6 (IL-6) fixés sur une microplaque. Après rinçage, un anticorps anti-interleukines-6 biotinylé est ajouté et se fixe spécifiquement. Une solution de péroxidase est ajoutée, se lie aux anticorps biotinylés puis réagit à l'ajout d'un substrat pour former une coloration bleue. La réaction est stoppée et une coloration jaune se forme dont l'intensité est proportionnelle à la concentration en interleukines 6. Parallèlement une courbe d'étalonnage en IL-6 est réalisée et sera exploitée pour déterminer la concentration en IL-6 dans les surnageants d'explants cutanés.

Dans le cadre de cette étude, chaque condition et/ou échantillon testé a été analysé en triplicat avec les explants et pour le dosage des IL-6.

*Résultats*

Gamme étalon

Pour cette étude une gamme d'étalonnage en interleukines 6 allant de 0,0 à 50,0 pg/mL a été réalisée. Les absorbances de la gamme étalon en interleukines 6 à 450nm sont présentés dans le tableau 12.

[Tableau 12]

| Gamme interleukines 6 (pg/mL) | | | | | | | | |
|---|---|---|---|---|---|---|---|---|
| | Blanc | NSB | 0,0 | 0,5 | 1,0 | 5,0 | 10,0 | 50,0 |
| DO 450nm | 0,000 | 0,096 | 0,572 | 0,107 | 0,266 | 1,012 | 0,949 | 2,377 |
| DO 450nm - NSB | - | 0,000 | 0,476 | 0,01 | 0,170 | 0,916 | 0,853 | 2,281 |
| NSB : sérum physiologique. | | | | | | | | |

Entre 0,5 et 5,0 pg/mL d'IL-6, le courbe d'étalonnage est linéaire. L'équation de cette droite a correspond à $y = 0,1954x-0,0578$

Mesure des échantillons

Pour la réalisation de cette étude deux témoins ont été réalisés : un témoin « basal » pour le dosage du stress basal et un témoin solvant, représentatifs du stress causé par la méthode et le solvant employés.

Les résultats d'absorbance (DO) à 450 nm des témoins et des échantillons sont présentés respectivement dans les tableaux 13 et 14, ci-dessous.

[Tableau 13]

| DO 450 nm des témoins | | | | | | |
|---|---|---|---|---|---|---|
| | Témoin basal | | | Témoin solvant | | |
| | Réplicat 1 | Réplicat 2 | Réplicat 3 | Réplicat 1 | Réplicat 2 | Réplicat 3 |
| DO 450nm | 0,711 | 1,377 | 1,086 | 0,306 | 0,710 | 0,447 |

**13**

(suite)

| DO 450 nm des témoins | | | | | | |
|---|---|---|---|---|---|---|
| | Témoin basal | | | Témoin solvant | | |
| | Réplicat 1 | Réplicat 2 | Réplicat 3 | Réplicat 1 | Réplicat 2 | Réplicat 3 |
| DO 450nm - NSB | 0,615 | 1,281 | 0,990 | 0,210 | 0,614 | 0,351 |

[Tableau 14]

| DO 450nm des échantillons de composition I1 | | |
|---|---|---|
| | Réplicat 1 | Réplicat 2 |
| DO 450nm | 0,712 | 0,708 |
| DO 450nm - NSB | 0,616 | 0,612 |

Concentrations en IL-6

Les concentrations en IL-6 des témoins et des échantillons ont été déterminées en utilisant l'équation de la courbe d'étalonnage et sont présentées respectivement dans les tableaux 15 et 16, ci-dessous :

[Tableau 15]

| DO 450 nm des témoins | | | | | | |
|---|---|---|---|---|---|---|
| | Témoin basal | | | Témoin solvant | | |
| | Réplicat 1 | Réplicat 2 | Réplicat 3 | Réplicat 1 | Réplicat 2 | Réplicat 3 |
| Concen tration en IL-6 (pg/mL) | 3,44* | 6,85 | 5,36 | 1,37 | 3,44* | 2,09 |
| Moyenne | 6,106 | | | 1,729 | | |
| Ecart - type | 1,055 | | | 0,510 | | |
| [0203] * valeurs exclues car statistiquement abherrantes, non interprétables | | | | | | |

[Tableau 16]

| DO 450nm des échantillons de composition I1 | | |
|---|---|---|
| | Réplicat 1 | Réplicat 2 |
| Concentration IL-6 (pg/mL) | 3,45 | 3,43 |
| Moyenne | 3,438 | |
| Ecart -type | 0.014 | |

Dans les conditions de l'étude, la concentration en IL-6 du témoin stress basal est de 6,106 pg/mL et du témoin stress induit par le solvant de 1,729 pg/mL.
La concentration en IL-6 moyenne des explants exposés à la composition selon l'invention I1 (0,008g) est de 3,438 pg/mL soit 43,7% d'inhibition de la réponse par rapport au témoin basal.

Conclusion :

Les explants exposés à l'échantillon « Composition selon l'invention I1 » ont une réponse inhibée par rapport au témoin basal ; à la dose de 0,008 g le produit testé inhibe de 43,7% la réponse IL-6 ; la « Composition selon l'invention I1 » présente un effet apaisant, relaxant ou anti-stress très significatif sur cet explant cutané et plus particulièrement sur l'épiderme exposé au stress environnemental.

**BIBLIOGRAPHIE**

1. Andre CM, Hausman JF, Guerriero G. Cannabis sativa: The plant of the thousand and one molecules. Front Plant Sci. 2016; 7: 19.

2. LaVigne JE, Hecksel R, Keresztes A, Streicher JM. Cannabis Sativa terpenes are cannbimetic and selectively enhance cannabinoid activity. Nature Scientific Reports. 2021; 11: 83232.

3. Russo EB. The Case for the Entourage Effect and Conventional Breeding of Clinical Cannabis: No "Strain," No Gain. Frontiers in Plant Science. Perspective. 2019; 9: 1-8.]

**Revendications**

**1.** Composition comprenant une solution contenant au moins un dérivé du chanvre et une émulsion de Pickering H/E, ledit dérivé du chanvre étant choisi parmi les macérats huileux de fleurs de chanvre, les cannabinoïdes, les terpènes et les flavonoïdes et l'émulsion de Pickering H/E contenant une phase aqueuse, une phase huileuse végétale et un phyllosilicate organomodifié.

**2.** Composition selon la revendication 1 comprenant une solution contenant au moins un macérat huileux de fleurs de chanvre et une émulsion de Pickering H/E contenant une phase aqueuse, une phase huileuse végétale et un phyllosilicate organomodifié.

**3.** Composition selon la revendication 2 dans laquelle la solution contenant au moins un macérat huileux de fleurs de chanvre comprend au moins du CBD en une quantité allant de 0,005% à 24,000%, de préférence de 0,01% à 5,00% en poids par rapport au poids total de la solution; du CBDA en une quantité allant de 0,005% à 24,000%, de préférence 0,010% à 5,000% en poids par rapport au poids total de la solution; du THC en une quantité allant de 0,001% à 30,000%, de préférence 0,010% à 1,000% en poids par rapport au poids total de la solution; du CBG en une quantité allant de 0,001% à 22,000%, de préférence 0,010% à 3,000% en poids par rapport au poids total de la solution et du CBGA en une quantité allant de 0,001% à 22,000%, de préférence 0,010% à 3,000% en poids par rapport au poids total de la solution.

**4.** Composition selon l'une quelconque des revendications 2 ou 3 **caractérisée en ce que** la solution contenant au moins un macérat huileux de fleurs de chanvre est présente en une teneur allant de 0,1 à 25%, de préférence allant de 1% à 5% et avantageusement 3,4% en poids par rapport au poids total de la composition.

**5.** Composition selon l'une quelconque des revendications 1 à 4 dans laquelle le phyllosilicate modifié est présent en une quantité allant de 3% à 20%, de préférence 4% à 10% et de manière préférée 5% à 8% en poids par rapport au poids total de la composition.

**6.** Composition selon l'une quelconque des revendications 1 à 5 dans laquelle la phase aqueuse de l'émulsion de Pickering est présente en une quantité allant de 51% à 90%, de préférence 55% à 70% et de manière préférée 58% à 65% en poids par rapport au poids total de la composition.

**7.** Composition selon l'une quelconque des revendications 1 à 6 dans laquelle la phase huileuse vegétale de l'émulsion de Pickering comprend au moins une huile végétale choisie dans le groupe formé par l'huile de chanvre, l'huile de tournesol, l'huile de colza, l'huile d'olive, l'huile de cameline, l'huile d'arachide, l'huile de coco, l'huile de pépin de raisin, l'huile de ricin, l'huile d'argan, l'huile de Djansang, l'huile de dattier du désert, l'huile de nigelle, l'huile de figue de barbarie, l'huile de macadamia, l'huile de soja, l'huile de palme et de palmier, l'huile de Tamanu, l'huile de sésame, l'huile de lin, l'huile de noix, l'huile de noisette, l'huile de baobab, l'huile de fruit de la passion, l'huile de noix du Brésil, l'huile d'hibiscus, l'huile de pépin de courge, l'huile de Luffa, l'huile de Carapa, l'huile d'Onagre, l'huile de bourrache, huile d'avocat, l'huile d'amande, l'huile d'argousier, l'huile de noyau d'abricot, l'huile de noyau de cerise, l'huile de pépin de pomme, l'huile de grenade, l'huile de Jojoba, l'huile de rose musquée, l'huile de prune, le beurre de karité, le beurre de cacao, le beurre de Kokum, le beurre de mangue, le beurre de moabi, le beurre de Karanja, le beurre de Tucuma, le beurre de Cupuaçu, le beurre de Buriti, le beurre de Murumuru, le beurre de Kombo, le beurre de Kpangnan, les triglycérides d'acides caprylique/ caprique.

8. Composition selon l'une quelconque des revendications 1 à 7 dans laquelle la phase huileuse de l'émulsion de Pickering est présente en une quantité allant de 5% à 40%, de préférence 10% à 30% et de manière préférée 15% à 25% en poids par rapport au poids total de la composition.

9. Composition selon l'une quelconque des revendications 1 à 7 comprenant, dans un milieu acceptable, au moins un agent cosmétique choisi parmi les agents humectants, les épaississants, les agents de texture, les émulsifiants, les agents dispersants, les agents moussants, les émolients, les conservateurs, les colorants, les extraits de plantes, les fibres végétales, les minéraux, les agents correcteurs de pH, les principes actifs et les parfums.

10. Composition selon l'une quelconque des revendications 1 à 9 **caractérisée en ce qu'**elle est destinée à être appliquée sur la peau.

11. Procédé de préparation de la composition selon l'une quelconque des revendications 1 à 10 comprenant au moins les étapes suivantes dans cet ordre :

preparer la phase aqueuse ;
ajouter le phyllosilicate organomodifié à la phase aqueuse et mélanger ;
ajouter la phase huileuse végétale au mélange obtenu et obtenir une émulsion de Pickering H/E ;
ajouter la solution contenant au moins un macérat huileux de fleurs de chanvre à l'émulsion de Pickering H/E.

12. Utilisation cosmétique, non thérapeutique, de la composition selon l'une quelconque des revendications 1 à 10 en tant qu'agent apaisant, relaxant, anti-stress, de la peau.

13. Procédé de traitement cosmétique, non thérapeutique de la peau, comprenant au moins une étape d'application sur la peau de la composition selon l'une quelconque des revendications 1 à 10.

Europäisches
Patentamt

European
Patent Office

Office européen
des brevets

# RAPPORT DE RECHERCHE EUROPEENNE

Numéro de la demande

EP 25 16 9666

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | US 10 603 344 B2 (CANOPY GROWTH CORP [CA]) 31 mars 2020 (2020-03-31) * colonne 8, ligne 49 - ligne 67 * * colonne 5, ligne 1 - ligne 20 * * colonne 7, ligne 55 - colonne 8, ligne 12 * * colonne 8, ligne 34 - ligne 36 * * revendications * | 1-13 | INV. A61K8/25 A61K8/26 A61K8/365 A61K8/73 A61Q19/00 A61K8/92 A61K8/06 |
| A,D | FR 2 976 503 A1 (EPHYLA [FR]) 21 décembre 2012 (2012-12-21) * page 1, ligne 1 - ligne 4 * * page 2, ligne 4 - ligne 7 * * page 3, ligne 13 - ligne 22 * * page 3, ligne 30 - ligne 31 * * page 9, ligne 6 - ligne 27 * * revendications * | 1-13 | |
| A | US 8 084 499 B2 (OOTAKE SOUICHIROU [JP]; IMORI YOSHIHISA [JP] ET AL.) 27 décembre 2011 (2011-12-27) * colonne 1, ligne 19 - ligne 27 * * colonne 1, ligne 48 - ligne 57 * * colonne 2, ligne 50 - ligne 65 * * page 3, ligne 15 - ligne 26 * * colonne 4, ligne 45 - ligne 63 * * colonne 5, ligne 19 - ligne 35 * * page 5, ligne 56 - ligne 65 * * exemples * * revendications * | 1-13 | DOMAINES TECHNIQUES RECHERCHES (IPC) A61K |
| A | EP 4 321 150 A1 (ALULA PEREGRINA TRADING COMPANY [SA]) 14 février 2024 (2024-02-14) * exemples * | 1-13 | |

-----

-----

-----

-/--

Le présent rapport a été établi pour toutes les revendications

| Lieu de la recherche | Date d'achèvement de la recherche | Examinateur |
|---|---|---|
| Munich | 8 août 2025 | Irwin, Lucy |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

.....................................................................................

& : membre de la même famille, document correspondant

EPO FORM 1503 03.82 (P04C02)

page 1 de 2

EP 4 635 471 A1

Europäisches
Patentamt

European
Patent Office

Office européen
des brevets

# RAPPORT DE RECHERCHE EUROPEENNE

Numéro de la demande

EP 25 16 9666

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | US 2016/346339 A1 (FINLEY CONSTANCE [US] ET AL) 1 décembre 2016 (2016-12-01) * exemple 3 * * revendications * ----- | 1-13 | |
| A | EP 3 349 857 B1 (CAPSUM [FR]) 28 juillet 2021 (2021-07-28) * alinéa [0124] - alinéa [0130] * ----- | 1-13 | |
| A | FR 3 134 519 A1 (FRANCE COSMEPHYL LAB [FR]) 20 octobre 2023 (2023-10-20) * alinéa [0001] * * alinéa [0032] - alinéa [0038] * * alinéa [0046] * * alinéa [0118] - alinéa [0119] * * revendications * ----- | 1-13 | |

DOMAINES TECHNIQUES
RECHERCHES (IPC)

Le présent rapport a été établi pour toutes les revendications

| Lieu de la recherche | Date d'achèvement de la recherche | Examinateur |
|---|---|---|
| Munich | 8 août 2025 | Irwin, Lucy |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

.........................................................................................
& : membre de la même famille, document correspondant

EPO FORM 1503 03.82 (P04C02)

page 2 de 2

18

**EP 4 635 471 A1**

## ANNEXE AU RAPPORT DE RECHERCHE EUROPEENNE
## RELATIF A LA DEMANDE DE BREVET EUROPEEN NO.

EP 25 16 9666

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

08-08-2025

| Document brevet cité au rapport de recherche | Date de publication | Membre(s) de la famille de brevet(s) | Date de publication |
|---|---|---|---|
| US 10603344 B2 | 31-03-2020 | DE 102007046086 A1 | 09-04-2009 |
| | | EP 2192911 A2 | 09-06-2010 |
| | | EP 3446697 A1 | 27-02-2019 |
| | | ES 2692875 T3 | 05-12-2018 |
| | | PL 2192911 T3 | 31-05-2019 |
| | | RU 2010116181 A | 10-11-2011 |
| | | RU 2016121857 A | 29-11-2018 |
| | | US 2010216872 A1 | 26-08-2010 |
| | | US 2013012575 A1 | 10-01-2013 |
| | | US 2015044315 A1 | 12-02-2015 |
| | | US 2018125905 A1 | 10-05-2018 |
| | | US 2020155628 A1 | 21-05-2020 |
| | | WO 2009039843 A2 | 02-04-2009 |
| FR 2976503 A1 | 21-12-2012 | AUCUN | |
| US 8084499 B2 | 27-12-2011 | CN 101415641 A | 22-04-2009 |
| | | JP 5218046 B2 | 26-06-2013 |
| | | JP WO2007114463 A1 | 20-08-2009 |
| | | TW 200808653 A | 16-02-2008 |
| | | US 2009098169 A1 | 16-04-2009 |
| | | WO 2007114463 A1 | 11-10-2007 |
| EP 4321150 A1 | 14-02-2024 | AU 2023323190 A1 | 20-02-2025 |
| | | CN 119923249 A | 02-05-2025 |
| | | EP 4321150 A1 | 14-02-2024 |
| | | EP 4568649 A1 | 18-06-2025 |
| | | IL 318857 A | 01-04-2025 |
| | | KR 20250043548 A | 28-03-2025 |
| | | MA 71731 A | 30-05-2025 |
| | | WO 2024033784 A1 | 15-02-2024 |
| US 2016346339 A1 | 01-12-2016 | AU 2016211314 A1 | 17-08-2017 |
| | | AU 2021203916 A1 | 08-07-2021 |
| | | CA 2974292 A1 | 04-08-2016 |
| | | EP 3250200 A1 | 06-12-2017 |
| | | MX 386351 B | 18-03-2025 |
| | | US 2016346339 A1 | 01-12-2016 |
| | | US 2020054701 A1 | 20-02-2020 |
| | | US 2022031781 A1 | 03-02-2022 |
| | | US 2024033312 A1 | 01-02-2024 |
| | | WO 2016123475 A1 | 04-08-2016 |
| EP 3349857 B1 | 28-07-2021 | CN 108136219 A | 08-06-2018 |
| | | EP 3349857 A1 | 25-07-2018 |
| | | FR 3041252 A1 | 24-03-2017 |

Pour tout renseignement concernant cette annexe : voir Journal Officiel de l'Office européen des brevets, No.12/82

page 1 de 2

## ANNEXE AU RAPPORT DE RECHERCHE EUROPEENNE
## RELATIF A LA DEMANDE DE BREVET EUROPEEN NO.

EP 25 16 9666

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

08-08-2025

| Document brevet cité au rapport de recherche | Date de publication | Membre(s) de la famille de brevet(s) | | Date de publication |
|---|---|---|---|---|
| | | US | 2019060186 A1 | 28-02-2019 |
| | | WO | 2017046305 A1 | 23-03-2017 |
| FR 3134519 A1 | 20-10-2023 | FR | 3134519 A1 | 20-10-2023 |
| | | WO | 2023198800 A1 | 19-10-2023 |

Pour tout renseignement concernant cette annexe : voir Journal Officiel de l'Office européen des brevets, No.12/82

page 2 de 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2016123475 A

- FR 2976503

**Littérature non-brevet citée dans la description**

- **ANDRE CM ; HAUSMAN JF ; GUERRIERO G.** Cannabis sativa: The plant of the thousand and one molecules. *Front Plant Sci.*, 2016, vol. 7, 19
- **LAVIGNE JE ; HECKSEL R ; KERESZTES A ; STREICHER JM**. Cannabis Sativa terpenes are cannbimetic and selectively enhance cannabinoid activity. *Nature Scientific Reports*, 2021, vol. 11, 83232

- **RUSSO EB**. The Case for the Entourage Effect and Conventional Breeding of Clinical Cannabis: No "Strain," No Gain. *Frontiers in Plant Science. Perspective*, 2019, vol. 9, 1-8